(19) Europäisches Patentamt
European Patent Office
Office européen des brevets

(11) Numéro de publication: **0 161 163**
**B1**

(12) **FASCICULE DE BREVET EUROPÉEN**

(45) Date de publication du fascicule du brevet: **23.09.87**

(51) Int. Cl.⁴: **C 07 D 335/00**, C 07 D 409/06

(21) Numéro de dépôt: **85400695.4**

(22) Date de dépôt: **09.04.85**

(54) **Dérivés de l'hydroxy-4-2H-1-benzothiopyran-2-one, leurs préparations et applications.**

(30) Priorité: **12.04.84 FR 8405794**

(43) Date de publication de la demande: **13.11.85 Bulletin 85/46**

(45) Mention de la délivrance du brevet: **23.09.87 Bulletin 87/39**

(84) Etats contractants désignés: **AT BE CH DE FR GB IT LI LU NL SE**

(56) Documents cité: **FR-A-2 351 653**

(73) Titulaire: **LIPHA, LYONNAISE INDUSTRIELLE PHARMACEUTIQUE, 34, rue Saint Romain Boîte Postale 8481, F-69359 Lyon Cedex 08 (FR)**

(72) Inventeur: **Berthelon, Jean-Jacques, 1, allée des Glycines, F-69150 Decines-Charpieu (FR)**

(74) Mandataire: **Bouton Neuvy, Liliane, L'Air liquide, Société Anonyme pour L'Etude et L'Exploitation des Procédés Georges Claude 75, Quai d'Orsay, F-75321 Paris Cedex 07 (FR)**

**EP 0 161 163 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne des hydroxy-4-2H-1-benzothiopyran-2-ones de formule (I), leur préparation et les compositions les contenant.

On connaît l'intérêt de certains dérivés thio-1 hydroxy-4 coumarine, notamment par le brevet FR 2.351.653, qui a pour objet les composés repondant à la formule générale

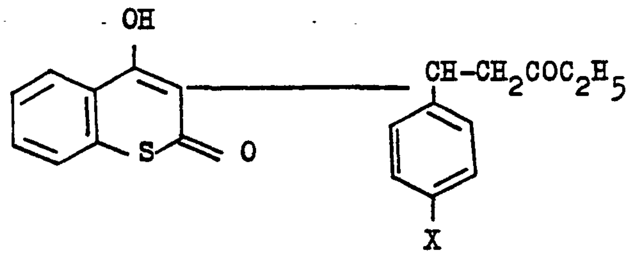

dans laquelle X désigne un atome d'hydrogène, de chlore ou de brome ou un groupe nitro; et le médicament à activité anticoagulante dans lequel le principe actif constitué par un dérivé de thio-1 hydroxy-4 coumarine est associé à un véhicule thérapeutiquement administrable.

Il a été recherché des composés nouveaux, notamment substitués par un groupement biphényle ou phénoxyphényle, à activité anticoagulante, susceptibles de constituer le principe actif de composition rodenticide, permettant l'application d'une thérapeutique antidotale vis à vis des animaux non rongeurs.

Ces composés sont représentés par la formule:

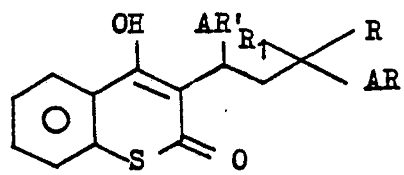

(I)

dans laquelle: R peut former avec AR' un cycle tétrahydronaphtyle, quand $R_1$ est l'hydrogène; R et $R_1$ peuvent être un groupement carbonyle avec l'atome de carbone adjacent; R peut être un groupement hydroxyle quand $R_1$ est l'hydrogène; AR est un groupement biphényle ou phénoxyphényle éventuellement substitué par un halogène; et AR' est un groupement phényle ou thiényle ou peut former avec R un cycle tétrahydronaphtyle, quand $R_1$ est l'hydrogène.

Ils peuvent se préparer selon l'une ou l'autre des méthodes ci-après.

Quand dans la formule (I) $RR_1 = 0$, les produits sont obtenus en faisant réagir l'hydroxy-4-2H-1-benzothiopyran-2-one avec des composés de formule:

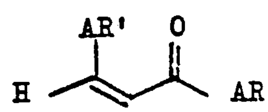

(II)

dans un solvant comme l'éthanol le dioxanne, en présence d'un agent de condensation comme la pipéridine à l'ébullition pendant plusieurs heures.

Quand dans la formule (I) R = H et $R_1$ = OH, les composés sont obtenus en réduisant à l'aide de borohydrure de sodium, dans un solvant tel le méthanol, le diméthylformamide ou le mélange de l'un de ces solvants avec de l'eau, le composé de formule (I) dans lequel $RR_1 = 0$.

Dans le cas où R et AR' forment un cycle tétrahydronaphtyle et $R_1$ est l'hydrogène, les composés répondant à la formule (I) sont préparés en faisant réagir l'hydroxy-4-2H-1-benzothiopyran-2-one avec un composé de formule

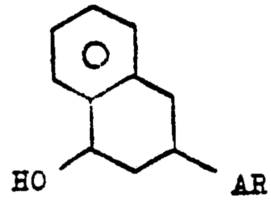

(III)

dans un solvant tel que l'acide acétique en présence d'acide sulfurique et entre 20° C et 150° C.

Lorsque $R_1$ = OH ou lorsque R et AR' forment un cycle tétrahydronaphtyle et $R_1$ est l'hydrogène, les composés se présentent sous la forme d'un mélange de diastéréoisomères, comme ceci est montré en chromatographie en couche mince ou en chromatographie liquide haute pression.

Les produits de l'invention possèdent d'intéressantes propriétés en particulier comme anticoagulants, comme le montrent les résultats des essais effectués chez le rat sauvage (Rattus Norvegicus).

Pour cela un appât dosé à 50 ppm en substance à examiner est préparé par imprégnation de blé vaseliné avec un mélange constitué d'amidon et du composé étudié. Cet appât est ensuite donné pendant 3 jours à des rats sauvages mâles ou femelles adultes, et l'activité est examinée en déterminant la mortalité. Les résultats obtenus sont indiqués dans le tableau I ci-dessous.

**Tableau I**

Activité sur Rattus Norvegicus (souche sensible au Coumafène)

| Composé | Dose | Durée du traitement | Mortalité |
|---|---|---|---|
| 1 | 50 ppm | 3 jours | 10/10 |
| 2 | 50 ppm | 3 jours | 10/10 |
| 3 | 50 ppm | 3 jours | 10/10 |
| 4 | 50 ppm | 3 jours | 10/10 |
| 5 | 50 ppm | 3 jours | 10/10 |
| 6 | 50 ppm | 3 jours | 10/10 |
| 7 | 50 ppm | 3 jours | 10/10 |
| 8 | 50 ppm | 3 jours | 10/10 |

[Coumafène = Warfarine = hydroxy-4-(phényl-1-oxo-3 butyl)-3-2H-1 benzopyran-2-one].

Les composés ont également montré que dans le cas d'une prise unique, ils conduisaient à une mortalité de 100 % sur Rattus Norvegicus, comme ceci est indiqué dans le tableau II suivant.

**Tableau II**

Activité sur Rattus Norvegicus (souche sensible au Coumafène)

| Composé | Dose | Durée | Mortalité |
|---|---|---|---|
| 1 | 50 ppm | 1 jour | 10/10 |
| 2 | 50 ppm | 1 jour | 10/10 |
| 3 | 50 ppm | 1 jour | 10/10 |

D'autre part il a été constaté que les dérivés thiopyranniques (I) de l'invention sont supérieurs aux dérivés pyranniques correspondants, résultat qui présente un caractère inattendu. En effet, il est connu que les dérivés de l'hydroxy-4 coumarine substitués en position 3 sont de puissants antivitaminiques K et sont utilisés comme rodenticides. Les travaux de Mentzer, Bull. Soc. Chim. Biol. 25, pages 379-383 (1943) ont d'autre part montré que le remplacement de l'oxygène hétérocyclique du dicoumarol par le soufre conduisait à un produit très peu actif. Le résultat a été confirmé et d'autre part, ceci a été également vérifié pour le coumafène et son homologue soufré le thiocoumafène. Le tableau III ci-dessous regroupe ces résultats.

**Tableau III**

Activité sur Rattus Norvegicus (souche sensible au Coumafène)

| Composé | Dose | Durée | Mortalité |
|---|---|---|---|
| coumafène | 250 ppm | 3 jours | 8/10 |
| thiocoumafène | 250 ppm | 3 jours | 5/10 |

Le composé préféré selon l'exemple 1, comparé à son équivalent oxygéné (A) le [(bromo-4'-biphényl-4)-3, tétrahydro-1-2-3-4-naphtyl-1]-3, hydroxy-4-2H-[1]-benzopyran-2-one a une activité nettement supérieure à celui-ci. Les essais comparatifs ont été effectués sur: Rattus rattus (souche résistante au Coumafène); Mus musculus (souche sensible au Coumafène); et Mus musculus (souche résistante au Coumafène). Les résultats obtenus sont indiqués dans les trois tableaux suivants:

**Tableau IV**

| Essai sur Rattus Rattus (souche résistante au Coumafène) | | | |
|---|---|---|---|
| Composé | Dose | Durée d'administration | Mortalité |
| 1 | 5 ppm | 2 jours | 20/20 |
| A | 5 ppm | 2 jours | 11/20 |

**Tableau V**

| Essai sur Mus musculus (souche sensible au Coumafène) | | | |
|---|---|---|---|
| Composé | Dose | Durée d'administration | Mortalité |
| 1 | 5 ppm | 7 jours | 20/20 |
| A | 5 ppm | 7 jours | 15/20 |

**Tableau VI**

| Essai sur Mus musculus (souche résistante au Coumafène) | | | |
|---|---|---|---|
| Composé | Dose | Durée d'administration | Mortalité |
| 1 | 5 ppm | 7 jours | 10/10 |
| A | 5 ppm | 7 jours | 7/10 |

La lecture de ces tableaux indique que dans le cas des composés répondant à la formule (I), la présence du cycle benzothiopyrannique conduit à une activité supérieure par rapport aux analogues benzopyranniques.

Un autre effet inattendu obtenu est la moindre toxicité des composés de l'invention par rapport à leurs homologues oxygénés notamment sur les espèces non rongeurs. A titre d'exemple l'essai suivant a été conduit sur le chien de race Beagle:

3 chiens de race Beagle ont reçu 50 µg/kg du composé selon l'exemple 1, dit composé 1, chaque jour par voie orale et 3 chiens de race Beagle ont reçu la même dose du composé A selon le même protocole. La surveillance biologique a comporté la détermination du temps de Quick avec arrêt de l'essai lorsque celui-ci atteignait 300 secondes. Les courbes de la figure I du dessin annexé, visualisent les effets obtenus sur l'allongement du temps de Quick chez le chien Beagle, les temps de Quick en secondes sont portés en ordonnées (TQs) et la durée du traitement en jours (j) en abscisses. Les courbes 1, 2 et 3 correspondent aux effets du composé A et les courbes 4, 5 et 6 à ceux du composé 1. La lettre M désigne le moment de la mort d'un animal.

De plus, il a été observé que dans le cas des chiens traités par le composé A, la mort apparait très rapidement, dès l'augmentation du temps de Quick, rendant impossible l'application d'une thérapeutique antidotale. Par contre, pour le composé 1 de l'invention, l'augmentation du temps de Quick et la détérioration de l'état clinique sont progressife. Lorsque le temps de Quick atteint 300 secondes, on a le temps de procéder à l'application d'un traitement classique de sauvegarde à base de vitamine $K_1$: une injection I.V. de 5 mg/kg relayée par un traitement par voie orale. Ainsi les 3 chiens montrent rapidement un retour du temps de Quick vers des valeurs normales et ce définitivement.

Les composés de l'invention, et en particulier le composé de l'exemple; constituent des substances actives anticoagulantes de compositions rodenticides, en association avec un support consommable par les rongeurs.

Il est donné ci-après des exemples qui illustrent l'invention à titre non limitatif.

**Exemple 1: [(Bromo-4'-biphényl-4)-3, tétrahydro-1,2,3,4-naphtyl-1]-3, hydroxy-4-2H-[1]-benzothiopyran-one-2.**

$C_{31}H_{23}BrO_2S$ (formule 1)
P.M = 539,47.

On porte à 110°C une solution de 14,2 g (0,08 mole) d'hydroxy-4-2H-1-benzothiopyran-2-one et 30,2 g (0,08 mole) de (bromo-4'-biphényl-4)-3-tétrahydro-1,2,3,4-naphtol-1 dans 60 ml d'acide acétique. On ajoute ensuite 2,6 ml d'acide sulfurique concentré et on poursuit le chauffage pendant trois heures. Après avoir refroidi on verse le milieu réactionnel dans l'eau et on extrait à l'éther. La solution éthérée est lavée avec de la soude diluée et l'huile insoluble formée est décantée et reprise par l'acide chlorhydrique 10 N. Après extraction avec

l'acétate d'éthyle, évaporation et passage de l'huile obtenue sur une colonne de silice éluée au chloroforme, le solide obtenu est recristallisé dans le toluène. On obtient 9,5 g (rdt. 22 %) d'un solide blanc. γ C = O : 1600 - 1620 $cm^{-1}$.

| Analyse pondérale: | C % | H % | Br % | S % | O % |
|---|---|---|---|---|---|
| Calculée | 69,01 | 4,30 | 14,81 | 5,94 | 5,93 |
| Trouvée | 68,97 | 4,15 | 14,85 | 5,82 | |

Un examen en chromatographie en couche mince indique la présence en proportion variable de deux isomères, l'un étant majoritaire. Il en résulte au niveau du point de fusion une plage qui dans le cas du produit décrit est $PF_G$: 203-227° C. Par purification, il est possible de séparer les deux isomères. L'isomère majoritaire recristallisé dans le toluène fond à 227-230° C. L'isomère minoritaire fond à 209-211° C.

**Exemple 2: [(Bromo-4' phénoxy)-4-phényl)-3, tétrahydro-1,2,3,4-naphtyl-1]-3, hydroxy-4-2H-[1]-benzothiopyran-2-one.**

$C_{31}H_{23}BrO_3S$ (formule 2)
P.M. = 555,47.

Préparé selon l'exemple 1 à partir de 6 g (0,015 mole) de [(bromo-4' phénoxy-)-4-phényl]-3, tétrahydro-1,2,3,4-naphtol-1 et 2,7 g (0,015 mole) d'hydroxy-4-2H-1-benzothiopyran-2-one. On obtient après traitement à l'hexane un solide beige $PF_G$: 95-105° C. IR γC = O: 1595.

| Analyse pondérale: | C % | H % | Br % | O % | S % |
|---|---|---|---|---|---|
| Calculée | 67,03 | 4,17 | 14,39 | 8,64 | 5,77 |
| Trouvée | 67,22 | 4,23 | 14,09 | | 5,79 |

**Exemple 3: [(Chloro-4'-biphényl-4)-3, tétrahydro-1,2,3,4-naphtyl-1]-3, hydroxy-4-2H-[1]-benzothiopyran-one-2**

(formule 3) $C_{31}H_{23}ClO_2S$.
P.M. = 495,01.

Préparé selon l'exemple 1 à partir de 7 g (0,04 mole) d'hydroxy-4-2H-1-benzothiopyran-2-one et 13,4 g (0,04 mole) de (chloro-4'-biphényl-4)-3, tétrahydro-1,2,3,4-naphtol-1. On obtient après purification dans l'acétate d'éthyle un solide blanchâtre $PF_G$: 220 - 2° C, IR γC = O: 1590

| Analyse pondérale: | C % | H % | Cl % | O % | S % |
|---|---|---|---|---|---|
| Calculée | 75,21 | 4,68 | 7,16 | 6,46 | 6,48 |
| Trouvée | 75,19 | 4,72 | 7,02 | | 6,20 |

**Exemple 4: [(Bromo 4'-biphényl-4)-3, oxo-3, phényl-1] propyl-3, hydroxy-4-2H-[1]-benzothiopyran-one-2**

(formule 4) $C_{30}H_{21}BrO_3S$.
P.M. = 541,45.

On place 5 g (0,020 mole) d'hydroxy-4-2H-1-benzothiopyran-one-2 et 9,1 g (0,025 mole) de (bromo-4' biphényl-4)-3, phényl-1-propenone-3 dans 50 ml d'éthanol contenant 0,4 ml de pipéridine. On porte cette suspension au reflux et on ajoute la quantité de dioxanne nécessaire à l'obtention d'une solution. Le milieu est ensuite maintenu au reflux pendant 6 heures. Après refroidissement à température ambiante on essore l'insoluble et le filtrat est fortement refroidi. Le précipité formé est essoré et purifié par passage sur une colonne de silice puis est recristallisé dans le toluène. On obtient ainsi un solide beige clair. $PF_G$: 169 - 171° C. IR γC = 0: 1580.

| Analyse pondérale: | C % | H % | Br % | O % | S % |
|---|---|---|---|---|---|
| Calculée | 66,54 | 3,91 | 14,76 | 8,87 | 5,92 |
| Trouvée | 66,43 | 3,82 | 14,73 | | 5,80 |

**Exemple 5: [(Bromo-4'-biphényl-4)-3, hydroxy-3, phényl-1] propyl-3, hydroxy-4-2H-[1]-benzothiopyran-one-2**

(formule 5) $C_{30}H_{23}BrO_3S$.
P.M. = 543,46.
On place dans un réacteur de 250 ml 7,9 g (0,0146 mole) du produit de l'exemple 4, 70 ml d'éthanol, 1,7 g (0,043 mole) de soude en pastille. On porte l'ensemble à 30°C et on ajoute par petites fractions 1,1 g (0,0292 mole) de borohydrure de sodium. On laisse ensuite 2 heures à 30-35°C, on filtre et on verse le filtrat sur le mélange $H_2O$-HCl concentré (600 ml - 30 ml). On essore le précipité blanc formé que l'on purifie par passage sur colonne de silice en éluant avec le mélange cyclohexane-$CHCl_3$-MeOH: 125 - 75 - 25. $PF_G$: 110-120°C. IR-γC = 0 : 1600.

| Analyse pondérale: | C % | H % | Br % | O % | S % |
|---|---|---|---|---|---|
| Calculée | 66,30 | 4,27 | 14,71 | 8,83 | 5,90 |
| Trouvée | 66,25 | 4,28 | 14,82 | | 5,86 |

**Exemple 6:[(chloro-4'-biphényl-4)-3, oxo-3, phényl-1] propyl-3, hydroxy-4-2H-[1]-benzothiopyran-one-2**

(formule 6) $C_{30}H_{21}ClO_3S$.
P.M. = 496,99.
Préparé selon l'exemple 4 à partir de 8,7 g (0,0273 mole) de (chloro-4' biphényl-4)-3, phényl-1-propenone-3 et 5,3 (0,03 mole) d'hydroxy-4-2H-[1]-benzothiopyran-one-2. On obtient après purification dans le toluène un solide blanc. $PF_G$: 171-3°C, IR γC = 0: 1590.

| Analyse pondérale: | C % | H % | Cl % | O % | S % |
|---|---|---|---|---|---|
| Calculée | 72,50 | 4,26 | 7,13 | 9,66 | 6,45 |
| Trouvée | 72,76 | 4,37 | 7,25 | | 6,21 |

**Exemple 7: [(chloro-4'-biphényl-4)-3, hydroxy-3, phényl-1]propyl-3, hydroxy-4-2H-[1]-benzothiopyran-one-2**

(formule 7) $C_{30}H_{23}ClO_3S$.
P.M. = 499.
Préparé selon l'exemple 5 à partir de 8 g (0,0161 mole) du produit de l'exemple 6, 1,9 g (0,0474 mole) de soude en pastilles, 50 ml de diméthylformamide, 10 ml d'eau et 1,2 g (0,0322 mole) de borohydrure de sodium. On isole un produit beige. $PF_G$: 105-125°C (décomposition). IR γC = 0 : 1585.

| Analyse pondérale: | C % | H % | Cl % | S % | O % |
|---|---|---|---|---|---|
| Calculée | 72,20 | 4,65 | 7,11 | 6,42 | 9,62 |
| Trouvée | 72,24 | 5,01 | 7,17 | 6,21 | |

**Exemple 8: [(Bromo-4'-biphényl-4)-3, oxo-3, (thiényl-2)-1] propyl-3, hydroxy-4-2H-[1]-benzothiopyran-one-2**

(formule 8). $C_{28}H_{19}BrO_3S_2$.
P.M. = 547,47.
Prépare selon l'exemple 4 à partir de 11 g (0,03 mole) de (bromo-4'-biphényl-4)-3, (thiényl-2)-1-propenone-3 et 5,8 g (0,033 mole) d'hydroxy-4-2H-[1]-benzothiopyran-one-2. On obtient ainsi, par recristallisation dans l'acide acétique un solide blanc. $PF_G$ : 140-5°C. IR γC = 0 : 1600.

| Analyse pondérale: | C % | H % | Br % | S % | O % |
|---|---|---|---|---|---|
| Calculée | 61,42 | 3,50 | 14,60 | 11,71 | 8,77 |
| Trouvée | 61,65 | 3,52 | | 12,00 | |

**Revendications** pour les états contractants: BE, CH, DE; FR, GB, IT, LI, LU, NL, SE.

1. Hydroxy-4-2H-1-benzothiopyran-2-ones, caractérisés par la formule:

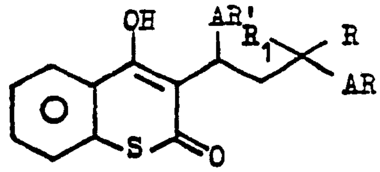

(I)

dans laquelle quand $R_1$ est l'hydrogène, R et AR' peuvent former un cycle tétrahydronaphtyle; R et $R_1$ peuvent former un groupement carbonyle avec l'atome de carbone adjacent; quand $R_1$ est l'hydrogène, R peut être un groupement hydroxyle; AR est un groupement biphényle ou phénoxyphényle éventuellement substitué par un halogène; AR' est un groupement phényle ou un thiényle ou peut former avec R un cycle tétrahydronaphtyle quand $R_1$ est l'hydrogène.

2. [(Bromo-4'-biphényl-4)-3, tétrahydro-1,2,3,4-naphtyl-1]-3, hydroxy-4-2H-[1]-benzothiopyran-one-2, selon la revendication 1.

3. Procédé de préparation des composés de la revendication 1, caractérisé par le fait qu'il consiste à faire réagir l'hydroxy-4-2H-1-benzothiopyran-2-one avec un composé de formule:

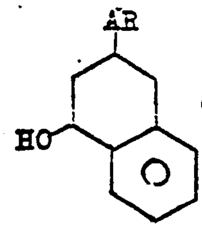

(III)

quand AR' et R forment un cycle tétrahydronaphtyle; ou avec un composé de formule générale:

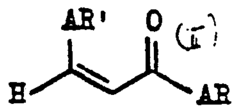

(II)

quand RR' forment un groupement carbonyle avec l'atome de carbon adjacent.

4. Procédé de préparation des composés de formule:

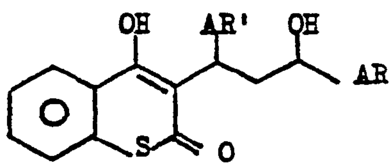

,

selon la revendication 1, caractérisé par le fait que ces composés sont obtenus par réduction des composée de formule:

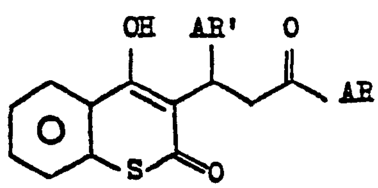

5. Composition rodenticide caractérisée en ce qu'elle contient comme substance active, un composé suivant la revendication 1 en association avec un support consommable par les rongeurs.

6. Composition rodenticide selon la revendication 5, caractérisée en ce qu'elle contient comme substance active, le [(bromo-4'-biphényl-4)-3, tétrahydro-1,2,3,4-naphtyl-1]-3, hydroxy-4-2H-[1]-benzothiopyran-one-2.

**Revendication** pour l'état contractant: AT.

1. Procédé de préparation des hydroxy-4-2H-1-benzothiopyran-2-ones représentés par la formule:

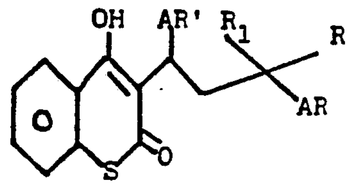

(I)

dans laquelle quand $R_1$ est l'hydrogène, R et AR' peuvent former un cycle tétrahydronaphtyle; R et $R_1$ peuvent former un groupement carbonyle avec l'atome de carbone adjacent; quand $R_1$ est l'hydrogène, R peut être un groupemennt hydroxyle; AR est un groupement biphényle ou phénoxyphényle éventuellement substitué par un halogène; AR' est un groupement phényle ou thiényle ou peut former avec R un cycle tétrahydronaphtyle quand $R_1$ est l'hydrogène, caractérisé en ce qu'il consiste à faire réagir l'hydroxy-4-2H-1-benzothiopyran-2-one avec un composé de formule:

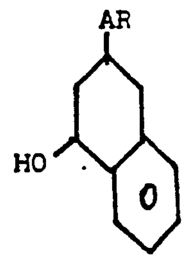

(III)

quand AR' et R forment un cycle tétrahydronaphtyle; ou avec un composé de formule générale:

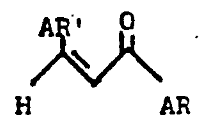

(II)

quand $RR_1$ forment un groupement carbonyle avec l'atome de carbone adjacent; puis quand R = H et $R_1$ = OH, ces composés sont obtenus par réduction des composés de formule:

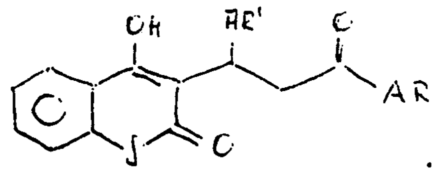

**Patentansprüche** für die Vertragsstaaten: BE, CH, DE, FR, GB, IT, LI, LU, NL, SE.

1. 4-Hydroxy-2H-1-benzothiopyran-2-one der Formel

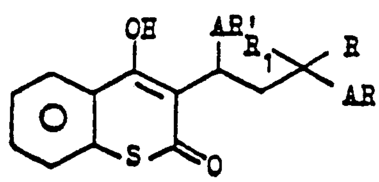

(I)

in welcher, wenn $R_1$ Wasserstoff ist, R und AR' einen Tetrahydronaphthylring bilden können; R und $R_1$ mit dem benachbarten Kohlenstoffatom eine Carbonylgruppe bilden können; wenn $R_1$ Wasserstoff ist, R eine Hydroxylgruppe sein kann; AR eine gegebenenfalls durch ein Halogen substituierte Biphenyl- oder Phenoxyphenylgruppe ist; und AR' eine Phenyl- oder eine Thienylgruppe ist oder mit R einen Tetrahydronaphthylring bilden kann, wenn $R_1$ Wasserstoff ist.

2. 3-[3-(4'-Brom-4-biphenyl)-1,2,3,4-tetrahydro-1-naphthyl]-4-hydroxy-2H-[1]-benzothiopyran-2-on nach Anspruch 1.

3. Verfahren zur Herstellung von Verbindungen nach Anspruch 1, dadurch gekennzeichnet, daß man 4-Hydroxy-2H-1-benzo-thiopyran-2-on mit einer Verbindung der Formel

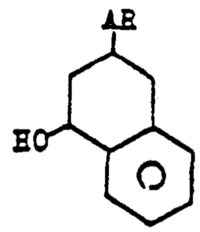

(III)

wenn AR' und R einen Tetrahydronaphthylring bilden, oder mit einer Verbindung der allgemeinen Formel

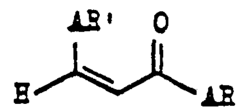

wenn RR' mit dem benachbarten Kohlenstoffatom eine Carbonylgruppe bildet, reagieren läßt.

4. Verfahren zur Herstellung von Verbindungen der Formel

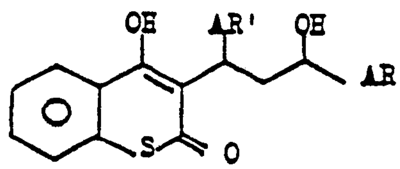

nach Anspruch 1, dadurch gekennzeichnet, daß diese Verbindungen durch Reduktion von Verbindungen der Formel

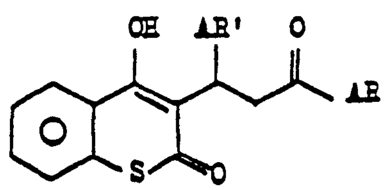

erhalten werden.

5. Rodentizides Mittel, dadurch gekennzeichnet, daß es als Wirkstoff eine Verbindung nach Anspruch 1 zusammen mit einem von Nagetieren verzehrbaren Träger enthält.

6. Rodentizides Mittel nach Anspruch 5, dadurch gekennzeichnet, daß es als Wirkstoff 3-[3-(4'-Brom-4-biphenyl)-1,2, 3,4-tetrahydro-1-naphthyl]-4-hydroxy-2H-[1]-benzothiopy-ran-2-on enthält.

**Patentansprüch**für den Vertragsstaat: AT.

Verfahren zur Herstellung von 4-Hydroxy-2H-1-benzothiopyran-2-onen der Formel

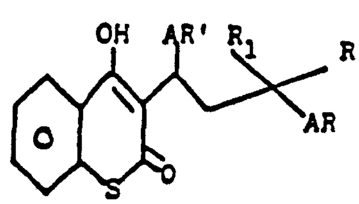

(I)

in welcher, wenn $R_1$ Wasserstoff ist, R und AR' einen Tetrahydronaphthylring bilden können; R und $R_1$ zusammen mit dem benachbarten Kohlenstoffatom eine Carbonylgruppe bilden können; wenn $R_1$ Wasserstoff ist, R eine Hydroxylgruppe sein kann; AR eine gegebenenfalls durch ein Halogen substituierte Biphenyl- oder

Phenoxyphenylgruppe ist; und AR' eine Phenyl- oder Thienylgruppe ist oder mit R einen Tetrahydronaphthylring bilden kann, wenn $R_1$ Wasserstoff ist, dadurch gekennzeichnet, daß man das 4-Hydroxy-2H-1-benzothiopyran-2-on mit einer Verbindung der Formel

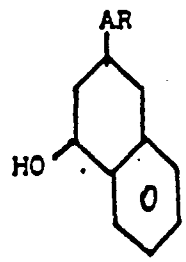

(III)

wenn AR' und R einen Tetrahydronaphthylring bilden, oder mit einer Verbindung der allgemeinen Formel

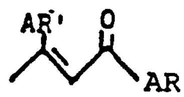

(II)

wenn $RR_1$ zusammen mit dem benachbarten Kohlenstoffatom eine Carbonylgruppe bildet, reagieren läßt und, wenn R = H und $R_1$ = OH, diese Verbindungen durch Reduktion von Verbindungen der Formel

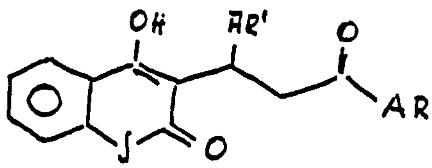

erhalten werden.

**Claims** for the contracting states: BE, CH, DE, FR, GB, IT, LI, LU, NL, SE

1. 4-Hydroxy-2H-1-benzothiopyran-2-ones characterized by the formula

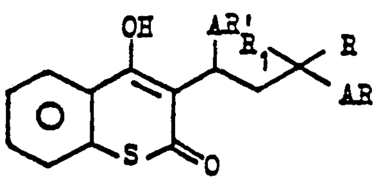

(I)

wherein, if $R_1$ is hydrogen, R and AR' may form a tetrahydronaphthyl ring; R and $R_1$ may form a carbonyl group together with the adjacent carbon atom; if $R_1$ is hydrogen, R may be a hydroxyl group; AR is a biphenyl or phenoxyphenyl group eventually substituted by a halogen; AR' is a phenyl or a thienyl group or may form a tetrahydronaphthyl ring with R, if $R_1$ is hydrogen.

2. 3-[3-(4'-bromo-4-biphenyl)-1,2,3,4-tetrahydro-1-naphthyl]-4-hydroxy-2H-[1]-benzothiopyran-2-one according to claim 1.

3. Process for preparing compounds according to claim 1, characterized in that one reacts the 4-hydroxy-2H-1-benzothiopyran-2-one with a compound of the formula

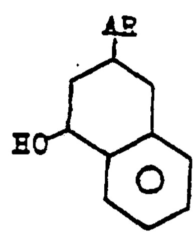

(III)

if AR' and R form a tetrahydronaphthyl ring, or with a compound of the general formula

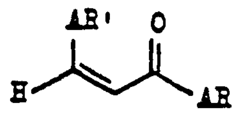

(II)

if RR' form a carbonyl group with the adjacent carbon atom.

4. Process for preparing compounds of the formula

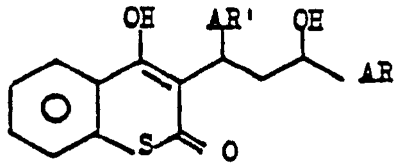

according to claim 1, characterized in that the said compounds are obtained by reduction of compounds of the formula

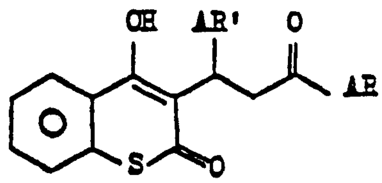

5. Rodenticide composition, characterized in that it contains as active substance a compound according to claim 1 in combination with a carrier consumable by the rodents.

6. Rodenticide composition according to claim 5, characterized in that it contains as active substance 3-[3-(4'-brommo-4-biphenyl)-1,2,3,4-tetrahydro-1-naphthyl ]-4-hydroxy-2H-[1]-benzothiopyran-2-one.

**Claim** for contracting state: AT.

Process for preparing 4-hydroxy-2H-1-benzothiopyran-2-ones of the formula

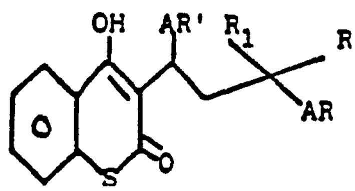

(I)

in which, if $R_1$ is hydrogen, R and RR' may form a tetrahydronaphthyl ring; R and $R_1$ may form a carbonyl group with the adjacent carbon atom; if $R_1$ is hydrogen, R may be a hydroxy group; AR is biphenyl or phenoxyphenyl group eventually substituted by a halogen; and AR' is a phenyl or thienyl group or may form a tetrahydronaphthyl ring with R, if $R_1$ is hydrogen, characterized in that one reacts the 4-hydroxy-2H-1-benzothiopyran-2-one with a compound of the formula

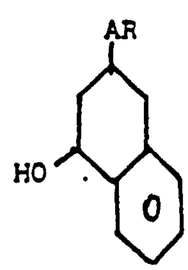

(III)

if AR' and R form a tetrahydronaphthyl ring, or with a compound of the general formula

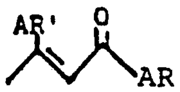

(II)

if $RR_1$ form a carbonyl group with the adjacent carbon atom, and, if R = H and $R_1$ = OH these compounds are obtained by reduction of the compounds of the formula

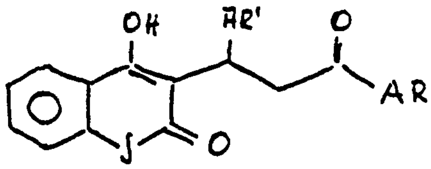

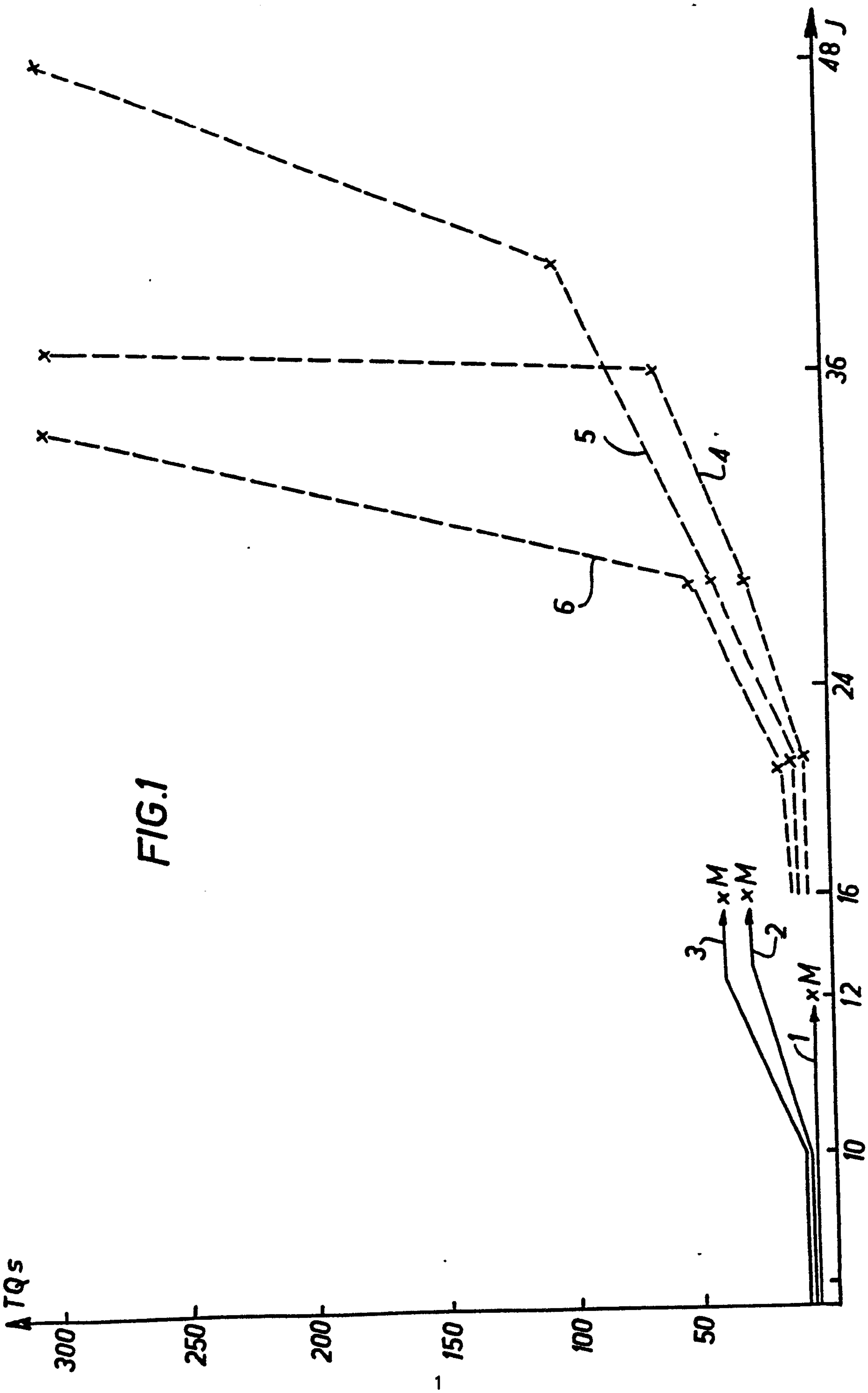


FIG.1

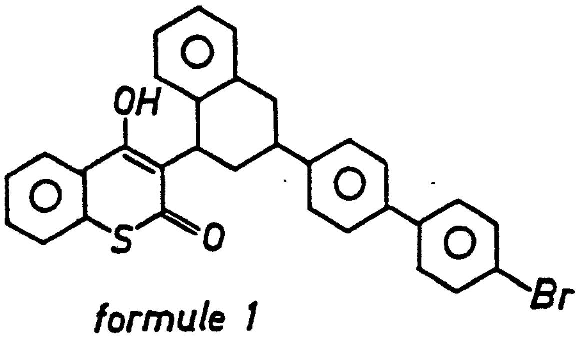

formule 1

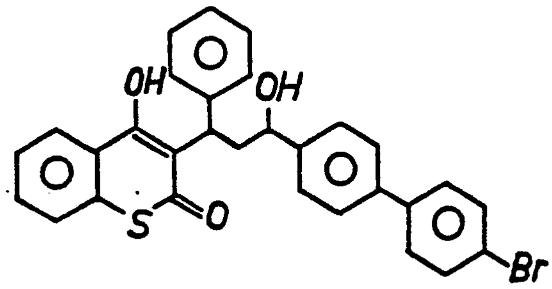

formule 5

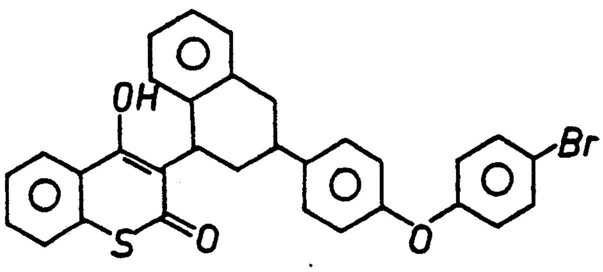

formule 2

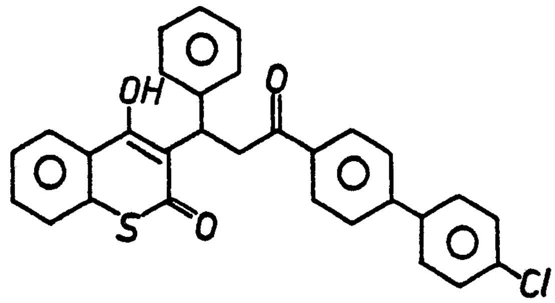

formule 6

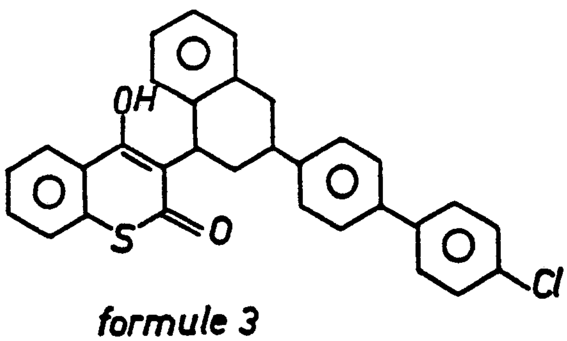

formule 3

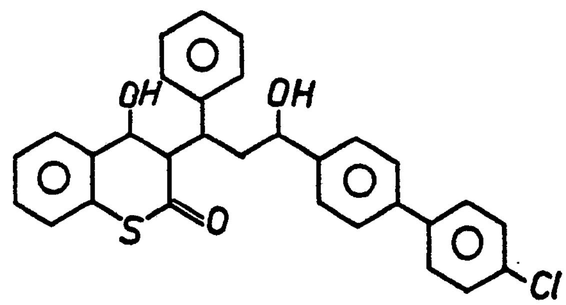

formule 7

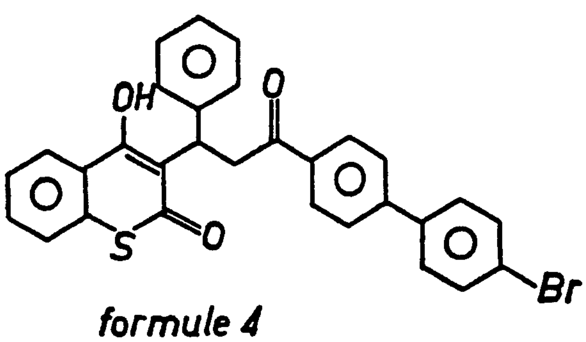

formule 4

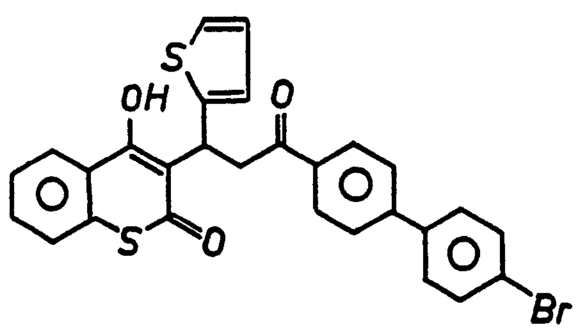

formule 8

FIG.2